# EUROPEAN PATENT SPECIFICATION

(11) **EP 4 286 120 B1**
(45) Date of publication and mention of the grant of the patent: **14.01.2026**
(21) Application number: 22871049.7
(22) Date of filing: 18.11.2022
(51) Int. Cl.: B65D 75/52, B65D 75/30

(54) **MANUFACTURING METHOD FOR MULTI-ROW DETECTION DEVICE**
HERSTELLUNGSVERFAHREN FÜR MEHRREIHIGE DETEKTIONSVORRICHTUNG
PROCÉDÉ DE FABRICATION D'UN DISPOSITIF DE DÉTECTION À RANGÉES MULTIPLES

(30) Priority: 08.04.2022 CN 202210370196; 26.04.2022 CN 202210442651
(43) Date of publication of application: 06.12.2023
(73) Proprietor: Leadway (HK) Limited, Sheung Wan, Hong Kong 999077 (CN)
(72) Inventor: WANG, Chunming, Hangzhou, Zhejiang 310030 (CN); SHANG, Tao, Hangzhou, Zhejiang 310030 (CN); CUI, Chao, Hangzhou, Zhejiang 310030 (CN); LING, Yun, Hangzhou, Zhejiang 310030 (CN); LI, Tianhao, Hangzhou, Zhejiang 310030 (CN)
(74) Representative: Schiweck Weinzierl Koch Patentanwälte Partnerschaft mbB
(86) International application number: PCT/CN2022/132929
(87) International publication number: WO 2023/193445

(56) References cited:
- EP-A2- 0 916 405
- CN-A- 103 057 765
- CN-A- 112 027 193
- CN-A- 114 536 470
- CN-U- 204 789 585
- CN-U- 213 081 605
- CN-Y- 2 329 621
- JP-A- 2008 239 194
- US-A1- 2009 151 864
- US-A1- 2012 080 353
- US-A1- 2018 209 971

## Description

### Field of the Invention

The present invention relates to a manufacturing method of a detection device, in particular to a manufacturing method of a multi-lined detection device, which is suitable for using a production process with high degree of automation.

### Background of the Invention

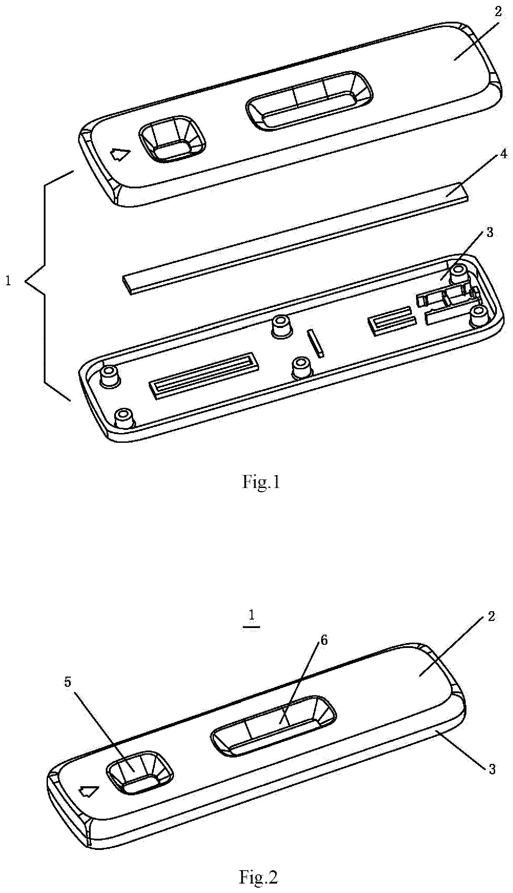

Disposable detection kits for detection of diseases or other physiological indicators using body fluids such as urine, blood or other tissue fluids of a human body have been commonly used all over the world, and their applications can be in laboratories operated by professionals, or at home, schools, shopping malls, road checkpoints, customs, etc. operated by non-professionals themselves. As shown in Fig. 1 and Fig. 2, such a detection kit 1 generally includes an upper cover 2, a bottom plate 3 and a test strip 4, wherein the upper cover 2 and the bottom plate 3 are combined in such a manner as buckling, welding, glue adhering or the like, and the test strip 4 is arranged on the bottom plate 3 and located between the upper cover 2 and the bottom plate 3.

The upper cover 2 includes a sample addition hole 5 and an observation hole 6. A liquid sample for detection is applied to the test strip 4 via the sample addition hole 5, then color change on the test strip 4 is observed via the observation hole 6 after the end of the detection, and the detection result is determined accordingly.

The upper cover 2 and the bottom plate 3 of such detection kit as shown in Fig. 1 and Fig. 2, are made of rigid plastics plates that are thermally injection molded, and they account for a large portion of the cost of the whole detection kit. Both the upper cover and the bottom plate have a certain height and width, and require additional aluminum foil bags for sealed packaging after their combination, so that the size of the whole detection kit 1 is large, and is much larger than the size of the test strip 4, increasing storage and transportation costs and increasing the technological process of production, thereby increasing production costs. Moreover, due to long degradation time of plastics, usage of a large amount of plastics does not comply with the concept of environmental protection. Furthermore, such detection kit is not suitable for manufacturing by production processes and equipment with high degree of automation, but requires a large number of workers for manual assembly, the assembly process is boring and cumbersome, and long time of operation is prone to resulting in fatigue, burnout and errors, reducing the qualification rate of products, and increasing labor costs.

As the disposable detection kits have been widely used, in order to reduce the purchase costs of users, it is necessary to reduce the price of products and improve the convenience of use as much as possible, so as to improve the usage intention of consumers. Particularly in the case of spreading of epidemic diseases, how to quickly promote such detection kits so that more people can afford and are willing to use such rapid detection kit products is a problem that needs to be solved at present.

A manufacturing method for test strips is known from US 2009/151864 A1.

An objective of the present invention is to provide a manufacturing method of a multi-lined detection device, by which automated production is easy to realize. The method is high in degree of automation, requires a small number of workers, has low labor intensity, produces products small in size, is low in production, packaging and transportation costs, and uses little plastic materials, so that it is very environmentally friendly.

### Summary of the Invention

In order to solve the above problems, the present invention provides a manufacturing method of a multi-lined detection device, the method comprising the following steps:
A, providing a bottom card band, a test strap and a sealing film band, wherein the thickness of the bottom card band is greater than the thickness of the sealing film band;
B, cutting the test strap into at least two test strips arranged side by side by using a cylindrical first hob, wherein the first hob is provided with at least two linear cutters on the cylindrical outer surface thereof, the linear cutters being parallel to the axis of the cylindrical outer surface and having equal distances from the axis of the cylindrical outer surface, the at least two linear cutters being arranged at equal intervals on the cylindrical outer surface of the first hob;
C, sequentially placing the cut test strips on the bottom card band such that the test strips are adhered to preset positions on the bottom card band;
D, transferring the sealing film band to the top of the bottom card band, bonding the sealing film band to the bottom card band by heating and squeezing using a heat sealing process, and packaging said at least two test strips respectively in corresponding airtight cavities between the sealing film band and the bottom card band to form a closed bonding band around each test strip; and
E, cutting the sealing film band and the bottom card band by using a cylindrical second hob to form incompletely disconnecting cutting lines and completely disconnecting cutting lines, the incompletely disconnecting cutting line connecting two adjacent detection devices, the completely disconnecting cutting line separating two adjacent detection devices, and the same number of at least two incompletely disconnecting cutting lines being disposed between every two adjacent completely disconnecting cutting lines, thereby forming the multi-lined detection device.

Further improvement of the present invention lies in that an elastic material is placed in an area between two adjacent linear cutters of the first hob, and the tips of the linear cutters exceed the elastic material by an appropriate distance.

Further improvement of the present invention lies in that the second hob is provided with at least two first rectangular cutters and second rectangular cutters equally spaced on the cylindrical outer surface thereof, and the same number of at least two first rectangular cutters are disposed between every two adjacent second rectangular cutters; the long sides of the first and second rectangular cutters are parallel to the axis of the cylindrical outer surface; and the first rectangular cutter forms two incompletely disconnecting cutting lines, and the second rectangular cutter forms one incompletely disconnecting cutting line and one completely disconnecting cutting line.

Further improvement of the present invention lies in that two long sides of the first rectangular cutter disposed on the cylindrical outer surface of the second hob are serrated blades, which form incompletely disconnecting cutting lines.

Further improvement of the present invention lies in that the serrated blades disposed on the cylindrical outer surface of the second hob completely cut off the sealing film band, but do not completely cut off the bottom card band or do not cut the bottom card band.

Further improvement of the present invention lies in that one of the long sides of each second rectangular cutter on the cylindrical outer surface of the second hob is a linear blade, the other of the long sides is a serrated blade, the linear blade forming an incompletely disconnecting cutting line, and the serrated blade forming an incompletely disconnecting cutting line.

Further improvement of the present invention lies in that the linear blade of the second rectangular cutter completely cuts off both the sealing film band and the bottom card band, and the serrated blade of the second rectangular cutter completely cuts off the sealing film band, but does not completely cut off the bottom card band or does not cut the bottom card band.

Further improvement of the present invention lies in that the thickness of the bottom card band ranges from 0.2mm to 1.5mm, and the thickness of the sealing film band ranges from 0.02mm to 0.1mm.

### Beneficial effects

As little plastic materials are used, the present invention is very environmentally friendly. The manufacturing method of the multi-lined detection device in the present invention is high in degree of automation and production efficiency, requires a small number of workers, has low labor intensity, and produces products small in size, so that the production, packaging and transportation costs are all much lower than those in the prior art. The product manufactured by using the present invention does not require additional aluminum foil bags for sealed packaging, which reduces the process steps, saves costs and lowers the price of products. In addition, the product manufactured with the present invention is small in size, portable, easy to operate and inexpensive.

Moreover, in the technical solution of the present invention that the sealing film band is completely cut off, when two adjacent detection devices are split, the tearing force cannot be transferred to the sealing film band of the adjacent detection devices because the two adjacent sealing film bands are completely disconnected, thus not separating the sealing film band of the adjacent detection devices from the bottom card band, so that packaging of the adjacent detection devices will not be affected.

Further, the present invention utilizes the same serrated blade, in the area where the incompletely disconnecting cutting line is formed, to completely cut off the sealing film band but not completely cut off the bottom card band in the same position and in one cutting step, thus greatly improving the production efficiency.

### Brief Description of the Drawings

Fig. 1 is a stereoscopic exploded view of an exsiting detection kit;
Fig. 2 is a stereoscopic schematic view of Fig. 1 after combination;
Fig. 3 is a schematic diagram of step B of a manufacturing method of a multi-lined detection device of the present invention;
Fig. 4 is a partially enlarged schematic view of a part of a cylindrical first hob shown in Fig. 3 within circle A;
Fig. 5 is a schematic diagram of step D of the manufacturing method of the multi-lined detection device of the present invention;
Fig. 6 is a schematic diagram of step E of the manufacturing method of the multi-lined detection device of the present invention;
Fig. 7 is a partially enlarged schematic view of a part within circle B in Fig. 6;
Fig. 8 is an enlarged schematic top view of a first rectangular cutter on a cylindrical second hob used in Fig. 6;
Fig. 9 is a sectional schematic view of Fig. 8 along line A-A;
Fig. 10 is an enlarged schematic top view of a second rectangular cutter on the cylindrical second hob used in Fig. 6;
Fig. 11 is a sectional schematic view of Fig. 10 along line B-B;
Fig. 12 is a top view of a multi-lined detection device produced using the manufacturing method of the present invention (the dashed line denotes the obscured part);
Fig. 13 is a sectional schematic view of Fig. 12 along line C-C;
Fig. 14 is a partially enlarged schematic view of a part within circle C in Fig. 13; and
Fig. 15 is a top view of a single detection device torn from the multi-lined detection device of Fig. 12.
Fig. 16 is a schematic diagram of side-by-side arrangement of a plurality of multi-lined detection devices produced by using the manufacturing method of the present invention.
Fig. 17 is a partially enlarged schematic view of a part within circle D in Fig. 16.
Fig. 18 is a view of an unfolded plane of the first and second rectangular cutters on the second hob.
Fig. 19 is an enlarged view of a part within circle E in Fig. 18.
Fig. 20 is a modified embodiment of Fig. 19.

### Detailed Description of the Embodiments

The technical solutions of the present invention will be further specified below through embodiments in conjunction with the drawings.

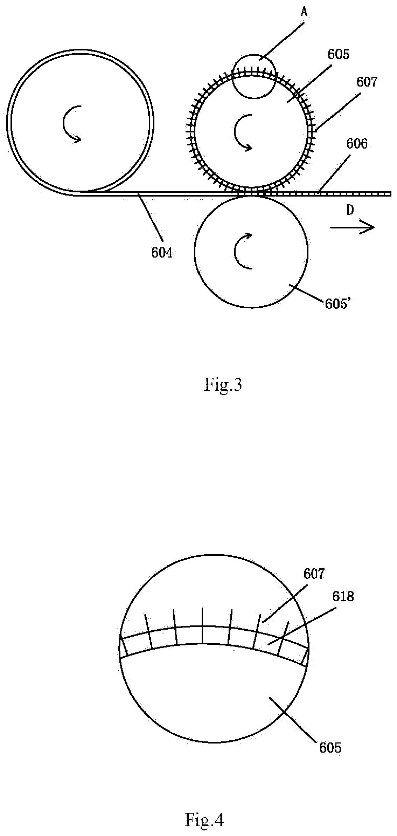

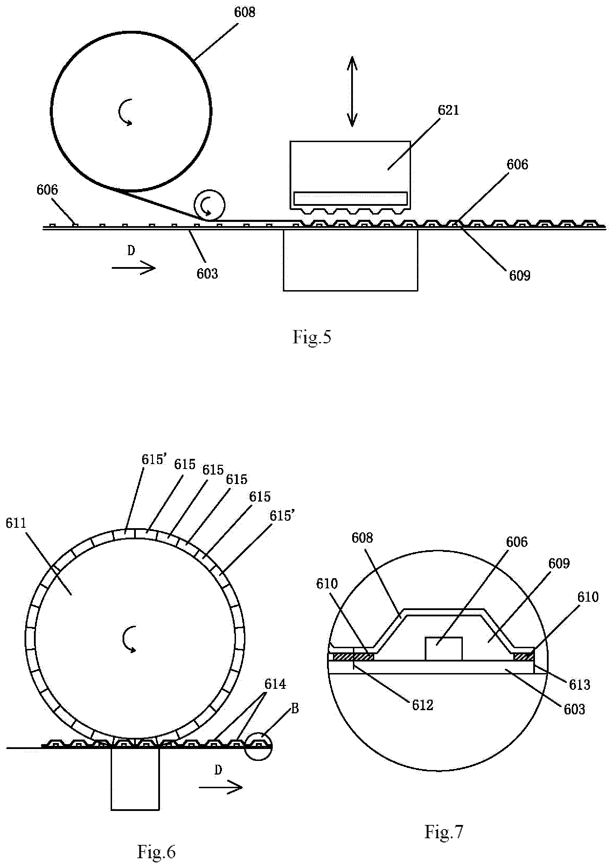

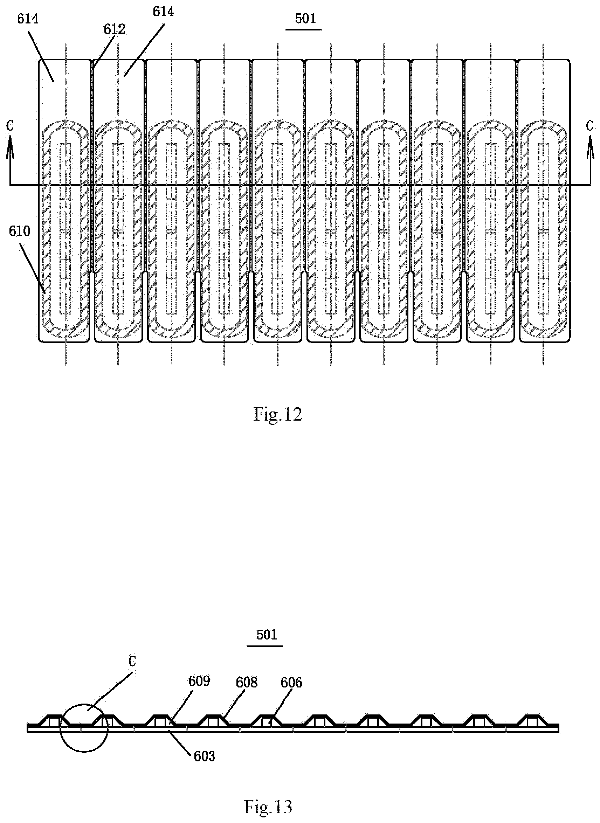

Please refer to Figs. 3 to 17, the present invention provides a manufacturing method of a multi-lined detection device, including the following steps: A, providing a bottom card band 603, a test strap 604 and a sealing film band 608, wherein the thickness of the bottom card band 603 is larger than that of the sealing film band 608; B, as shown in Fig. 3, a cylindrical first hob 605 is used to cooperate with a cylindrical pad wheel 605' to cut the test strap 604 into at least two test strips 606 arranged side by side, wherein the first hob 605 is provided with at least two linear blades 607 of sheet structures on the cylindrical outer surface thereof, and the cutting edges of the linear blades 607 are parallel to the axis of the cylindrical outer surface and have equal distances from the axis of the cylindrical outer surface, and the at least two linear blades 607 are arranged at equal intervals on the cylindrical outer surface of the first hob 605, so that the distance between the cutting edges of every two adjacent linear blades 607 is equal (the arrow "D" denotes the transfer direction of the test strap 604, the same in the full text); C, as shown in Fig. 5, the cut test strips 606 are sequentially placed on the bottom card band 603, and by means of, for example, mechanical arm gripping, negative pressure suction cup adsorption of the film to the upper surfaces of the test strips, manual placement of the test strips, etc., every two adjacent test strips 606 are equally spaced from each other at a preset distance and adhered to preset positions on the bottom card band; D, as shown in Fig. 5, the sealing film band 608 is transferred to the top of the bottom card band 603 where the test strips 606 are placed, and the sealing film band 608 is bonded to the bottom card band 603 by heating and squeezing (up and down movement as shown by arrows in Fig. 5) of a heat sealing mould 621 using a heat sealing process, and said at least two test strips 606 are packaged respectively in the corresponding airtight cavities 609 between the sealing film band 608 and the bottom card band 603 to form a closed bonding band 610 around each test strip; and E, as shown in Figs. 6 and 7, a cylindrical second hob 611 is used to cut the sealing film band 608 and the bottom card band 603 at the same time at the bonding band 610 to form incompletely disconnecting cutting lines 612 and completely disconnecting cutting lines 613, the incompletely disconnecting cutting line 612 connecting two adjacent detection devices 614, the completely disconnecting cutting line 613 separating the two adjacent detection devices 614, and the same number of at least two incompletely disconnecting cutting lines 612 being disposed between every two adjacent completely disconnecting cutting lines 613, thereby forming a multi-lined detection device 501 (as shown in Figs. 12, 13, 16 and 17).

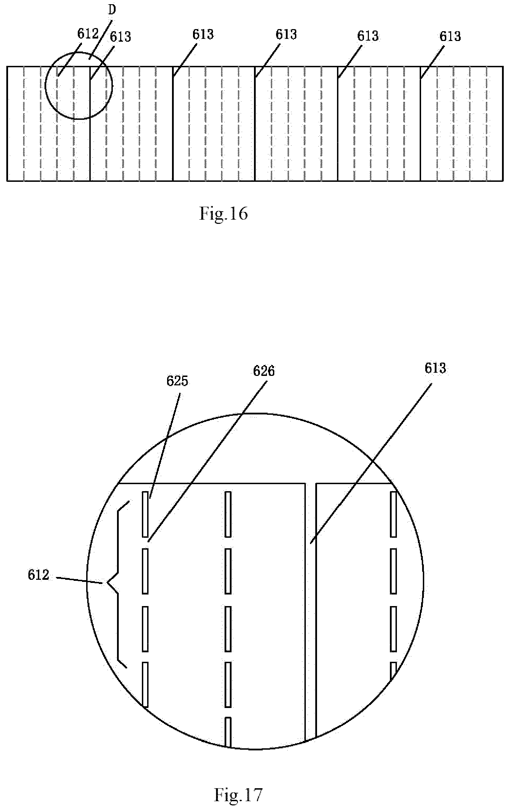

As shown in Fig. 6, the second hob 611 is provided with at least two rectangular cutters equally spaced on the cylindrical outer surface thereof, including first rectangular cutters 615 and second rectangular cutters 615', and at least two first rectangular cutters 615 are disposed between every two adjacent second rectangular cutters 615'. The long sides of the first and second rectangular cutters 615, 615' are parallel to the axis of the cylindrical outer surface, and the distance from the cutting edge of each long side to the axis of the cylindrical outer surface is equal. Completely disconnecting cutting lines 613 are formed by the second rectangular cutters 615', and the plurality of detection devices 614 between every two completely disconnecting cutting lines 613 form a multi-lined detection device 501. Incompletely disconnecting cutting lines 612 are formed by the first rectangular cutters 615, so that single detection devices 614 in the multi-lined detection device 501 are easy to separate. There are N first rectangular cutters 615 between every two adjacent second rectangular cutters 615', and then a multi-lined detection device 501 having N+1 single detection devices 614 is formed. For example in Fig. 6, there are four first rectangular cutters 615 between two second rectangular cutters 615', and the final multi-lined detection device has five single detection devices. Corresponding to this case, as shown in Fig. 16, four incompletely disconnecting cutting lines 612 (denoted by dashed lines in Fig. 16) are between every two adjacent completely disconnecting cutting lines 613 (indicated by solid lines in Fig. 16).

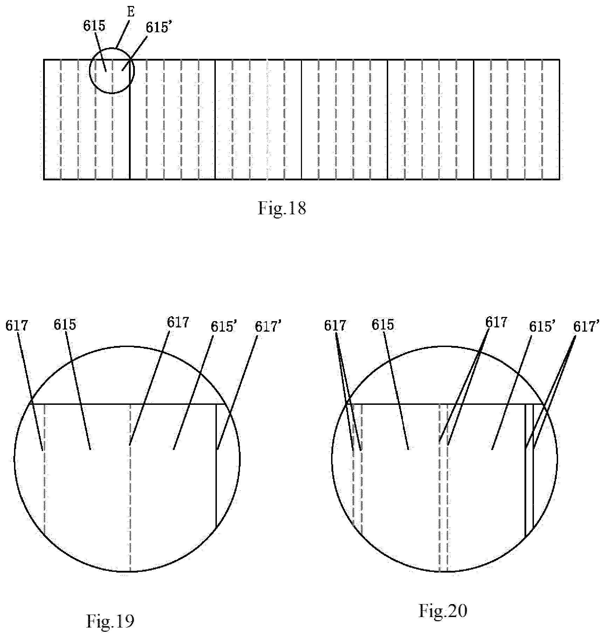

Fig. 18 is a view of an unfolded plane of the first and second rectangular cutters on the second hob, showing the arrangement mode of the first and second rectangular cutters. In one embodiment, two adjacent rectangular cutters (including the first rectangular cutter 615 and the second rectangular cutter 615') can share a long side 617 or 617' (as shown in Fig. 19). In another embodiment, two adjacent rectangular cutters (including the first rectangular cutter 615 and the second rectangular cutter 615') are completely independent, without sharing a long side. The cutters each have two long sides 617 or 617', and an appropriate gap is reserved between two adjacent long sides (as shown in Fig. 20).

Please refer to Fig. 4, an elastic material 618 is placed in an area between two adjacent linear blades 607 of the first hob 605, and the tips of the linear blades 607 exceed the elastic material 618 by an appropriate distance. The appropriate distance is smaller than the thickness of the test strip 606 to ensure that the test strip compresses the elastic material when being cut. The elastic material 618 is preferably rubber, foam, high-density sponge, silica gel, latex, etc. Placement of the elastic material 618 in the area between two adjacent linear blades 607 has the advantages that when the linear blades 607 are cutting the test strap 604, the elastic material 618 is compressed by the cut test strip 606, so that the notch is deeper; and after the linear blades 607 leave the cut test strip 606, the elastic material 618 returns to the original position to push out the test strip 606, solving the problem that the test strip 606 is clamped between the two adjacent linear blades 607 and thus is not easy to detach.

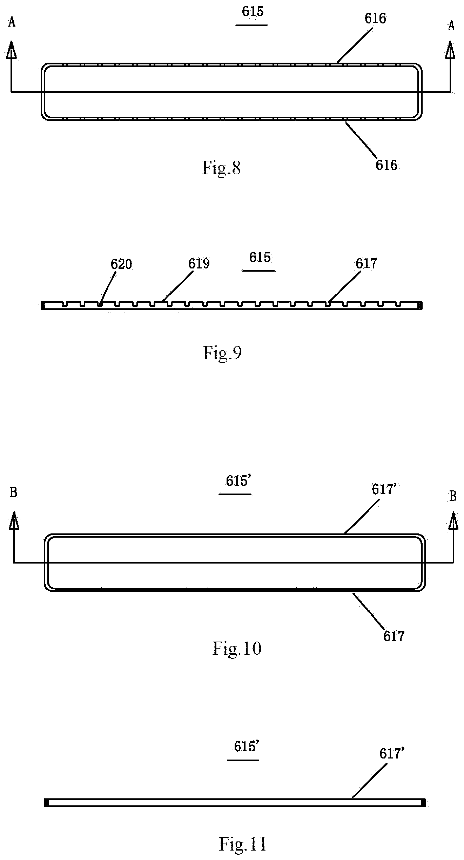

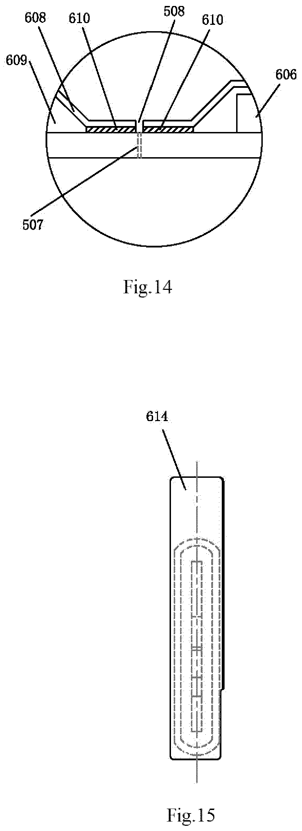

Please refer to Figs. 8 and 9, the two long sides 616 of the first rectangular cutter 615 on the cylindrical outer surface of the second hob 611 are serrated blades 617, which form an incompletely disconnecting cutting line 612 (as shown in Fig. 7), that is, the incompletely disconnecting cutting line 612 includes two technical solutions below: a, completely cutting off the sealing film band 608 and partially cutting the bottom card band 603 (for ease of separation of single detection devices); and b, completely cutting off the sealing film band 608 but not cutting the bottom card band 603 (for separating only the sealing film band of the single detection device for detection). The tooth portion 619 of the serrated blade 617 penetrates through the sealing film band 608 and the bottom card band 603 in an area between two adjacent bonding bands 610, thereby disconnecting the connection between the two adjacent bonding bands 610 in this area. Its advantage is reducing the resistance to tearing the incompletely disconnecting cutting lines 612. The serrated blade 617 completely cuts off only the sealing film band at the two bonding bands 610 through the cutting edge at the bottom of the recessed portion 620 to form a completely disconnected notch 508 (as shown in Fig. 14) without cutting the bottom card band, or further cuts away a part of the bottom card band located below the sealing film band after the sealing film band is completely cut off (including continuously or discontinuously cutting partial depth of the upper part of the bottom card band but not penetrating to the bottom surface of the bottom card band to form the incompletely disconnecting cutting line 612 as shown in Fig. 7, and discontinuously cutting the bottom card band and causing the cut portion to penetrate to the bottom surface of the bottom card band 603 to form the spaced cutting line 507 as shown in Fig. 14), to form an incompletely disconnecting cutting line 612 connecting two adjacent detection devices 614, thereby forming a multi-lined detection device 501 consisting of at least two detection devices 614. Preferably, the second hob as shown in Fig. 20 is selected, and the serrated blades 617 disposed on the cylindrical outer surface of the second hob 611 completely cut off the sealing film band 608 to form a completely disconnected notch 508 on the sealing film band 608 so as to form a gap, which is macroscopic or detachable by an instrument, between the cut-off sealing film bands (this gap is favorable for inspecting whether the sealing film band is completely cut off by naked eyes or an instrument, which contributes to improved qualification rate of products) , but do not completely cut off the bottom card band 603 (as shown in Fig. 14). That is, the bottom card band 603 is discontinously cut to form a the spaced cutting line 507 in the area cut on the bottom card band and form a connecting portion in the area not cut on the bottom card band. Furthermore, as shown in Fig. 17, the tooth portion 619 of the serrated blade 617 disposed on the cylindrical outer surface of the second hob 611 forms, on the bottom card band 603, the penetrative notch 625 penetrating through the bottom card band 603, while its recessed portion 620 does not cut the bottom card band 603, so as to form the connecting portion 626 spaced from the penetrative notch 625.

Please refer to Figs. 10 and 11, one of the long sides of the second rectangular cutter 615' disposed on the cylindrical outer surface of the second hob 611 is a serrated blade 617, while the other one is a linear blade 617' (i.e., its cutting edge is a straight line without serrations). The serrated blade 617 is as described previously, and the linear blade 617' completely cuts off both the sealing film band 608 and the bottom card band 603 to form a completely disconnecting cutting line 613 (as shown in Fig. 17), i.e., a side of the multi-lined detection device 501, thereby separating the multi-lined detection device 501 from the bottom card band 603 to obtain a product produced by using the manufacturing method of the present invention.

As shown in Figs. 12 and 13, it is the multi-lined detection device 501 produced by using the manufacturing method of the present invention. The multi-lined detection device 501 includes ten detection devices 614 arranged side by side, and every two adjacent detection devices are connected by an incompletely disconnecting cutting line 612. A bonding band 610 in an annular runway shape is formed for each detection device 614. Fig. 14 shows a structure of two adjacent detection devices 614 at a joint.

Fig. 15 shows a structure of a single detection device 614 torn from the multi-lined detection device 501, wherein the dashed line denotes the obscured part.

Fig. 16 shows a planar arrangement mode of a multi-lined detection device including five single detection devices, and particularly shows a planar arrangement mode of non-completely disconnecting cutting lines 612 and completely disconnecting cutting lines 613, that is, there are four non-completely disconnecting cutting lines 612 (shown by dashed lines in the figure) between two adjacent completely disconnecting cutting lines 613 (shown by solid lines in the figure).

The bottom card band 603 is preferably made of a moisture-proof and oxidation-resistant PE or PP material, with its thickness preferably ranging from 0.2mm to 1.5mm. The sealing film band 608 is preferably made of a moisture-proof and oxidation-resistant PE or PP or PET material, with its thickness preferably ranging from 0.02mm to 0.1mm. Since the thickness of the bottom card band 603 is much greater than the thickness of the sealing film band 608, the bottom card band 603 has moderate rigidity and higher strength, while the sealing film band 608 is softer and has better stretching resistance and tearing resistance performances, and both of them are suitable for producing inexpensive products on automated equipment by using the manufacturing method of the present invention.

Although Figs. 12 and 13 show a multi-lined detection device 501 including ten detection devices 614 arranged side by side, the number ten is variable and can be any suitable number such as two, five, twenty, etc., that is, the number of the detection devices 614 included in the multi-lined detection device produced by using the manufacturing method of the present invention can be set according to actual needs.

Moreover, in the technical solution of the present invention that the sealing film band is completely cut off, when two adjacent detection devices 614 are split, the tearing force cannot be transferred to the sealing film band 608 of the adjacent detection devices because the two adjacent sealing film bands 608 are completely disconnected, thus not separating the sealing film band 608 of the adjacent detection devices from the bottom card band 603, so that packaging of the adjacent detection devices 614 will not be affected.

Further, the present invention utilizes the same serrated blade, in the area where the incompletely disconnecting cutting line is formed, to completely cut off the sealing film band 608 but not completely cut off the bottom card band 603 in the same position and in one cutting step, thus greatly improving the production efficiency.

The above description is only the specific implementations of the present invention, and is not thereby limiting the scope claimed for protection by the present invention.

## Claims

1. A manufacturing method of a multi-lined detection device (501), comprising the following steps:
A, providing a bottom card band (603), a test strap (604) and a sealing film band (608), wherein the thickness of the bottom card band is greater than the thickness of the sealing film band;
B, cutting the test strap into at least two test strips (606) arranged side by side by using a cylindrical first hob (605), wherein the first hob is provided with at least two linear cutters (607) on the cylindrical outer surface thereof, the linear cutters being parallel to the axis of the cylindrical outer surface and having equal distances from the axis of the cylindrical outer surface, the at least two linear cutters being arranged at equal intervals on the cylindrical outer surface of the first hob;
C, sequentially placing the cut test strips on the bottom card band such that the test strips are adhered to preset positions on the bottom card band;
D, transferring the sealing film band to the top of the bottom card band, bonding the sealing film band to the bottom card band by heating and squeezing using a heat sealing process, and packaging said at least two test strips respectively in corresponding airtight cavities between the sealing film band and the bottom card band to form a closed bonding band (610) around each test strip; and
E, cutting the sealing film band and the bottom card band by using a cylindrical second hob (611) to form incompletely disconnecting cutting lines (612) and completely disconnecting cutting lines (613), the incompletely disconnecting cutting line connecting two adjacent detection devices, the completely disconnecting cutting line separating two adjacent detection devices, and the same number of at least two incompletely disconnecting cutting lines being disposed between every two adjacent completely disconnecting cutting lines, thereby forming the multi-lined detection device.

2. The manufacturing method of the multi-lined detection device of claim 1, wherein an elastic material is placed on the cylindrical outer surface of the first hob between two adjacent linear cutters of the first hob, and the tips of the linear cutters exceed the elastic material by an appropriate distance, wherein the appropriate distance is preferably smaller than the thickness of the test strip.

3. The manufacturing method of the multi-lined detection device of claim 1, wherein the second hob is provided with at least two first rectangular cutters and second rectangular cutters equally spaced on the cylindrical outer surface thereof, and the same number of at least two first rectangular cutters are disposed between every two adjacent second rectangular cutters; the long sides of the first and second rectangular cutters are parallel to the axis of the cylindrical outer surface; and the first rectangular cutter forms two incompletely disconnecting cutting lines, and the second rectangular cutter forms one incompletely disconnecting cutting line and one completely disconnecting cutting line.

4. The manufacturing method of the multi-lined detection device of claim 3, wherein two long sides of the first rectangular cutter disposed on the cylindrical outer surface of the second hob are serrated blades, which form incompletely disconnecting cutting lines.

5. The manufacturing method of the multi-lined detection device of claim 4, wherein the serrated blades disposed on the cylindrical outer surface of the second hob completely cut off the sealing film band, but do not completely cut off the bottom card band or do not cut the bottom card band.

6. The manufacturing method of the multi-lined detection device of claim 3, wherein one of the long sides of each second rectangular cutter on the cylindrical outer surface of the second hob is a linear blade, the other of the long sides is a serrated blade, the linear blade forming a completely disconnecting cutting line, and the serrated blade forming an incompletely disconnecting cutting line.

7. The manufacturing method of the multi-lined detection device of claim 6, wherein the linear blade of the second rectangular cutter completely cuts off both the sealing film band and the bottom card band, and the serrated blade of the second rectangular cutter completely cuts off the sealing film band, but does not completely cut off the bottom card band or does not cut the bottom card band.

8. The manufacturing method of the multi-lined detection device of claim 1, wherein the thickness of the bottom card band ranges from 0.2mm to 1.5mm, and the thickness of the sealing film band ranges from 0.02mm to 0.1mm.

9. The manufacturing method of the multi-lined detection device of claim 7, wherein the thickness of the bottom card band ranges from 0.2mm to 1.5mm, and the thickness of the sealing film band ranges from 0.02mm to 0.1mm.

## Patentansprüche

1. Herstellungsverfahren für eine multi-linierte Detektionsvorrichtung (501), umfassend die folgenden Schritte:
A, Bereitstellen eines unteren Kartenbandes (603), eines Teststreifens (604) und eines Siegelfolienbandes (608), wobei die Dicke des unteren Kartenbandes größer ist als die Dicke des Siegelfolienbandes;
B, Schneiden des Teststreifens unter Verwendung eines zylindrischen ersten Wälzfräsers (605) in mindestens zwei nebeneinander angeordnete Teststreifen (606) zu schneiden, wobei der erste Wälzfräser mit mindestens zwei Linearschneidern (607) auf der zylindrischen Außenfläche versehen ist, wobei die Linearschneider parallel zur Achse der zylindrischen Außenfläche sind und gleiche Abstände von der Achse der zylindrischen Außenfläche aufweisen, wobei mindestens zwei Linearschneider in gleichen Abständen auf der zylindrischen Außenfläche des ersten Wälzfräsers angeordnet sind;
C, sequentielles Platzieren der geschnittenen Teststreifen auf dem unteren Kartenband, so dass die Teststreifen an voreingestellten Positionen auf dem unteren Kartenband haften;
D, Übertragen des Siegelfolienbandes auf die Oberseite des unteren Kartenbandes, Kleben des Siegelfolienbandes an das untere Kartenband durch Erhitzen und Zusammendrücken unter Verwendung eines Heißsiegelverfahrens, und Verpacken der mindestens zwei Teststreifen jeweils in entsprechenden luftdichten Hohlräumen zwischen dem Siegelfolienband und dem unteren Kartenband, um ein geschlossenes Klebeband (610) um jeden Teststreifen zu bilden; und
E, Schneiden des Siegelfolienbandes und des unteren Kartenbandes unter Verwendung eines zylindrischen zweiten Wälzfräsers (611), um unvollständig trennende Schnittlinien (612) und vollständig trennende Schnittlinien (613) zu bilden, wobei die unvollständig trennende Schnittlinie zwei benachbarte Erkennungseinrichtungen verbindet, wobei die vollständig trennende Schnittlinie zwei benachbarte Detektionsvorrichtungen trennt, und wobei die gleiche Anzahl von mindestens zwei unvollständig trennenden Schnittlinien zwischen jeweils zwei benachbarten vollständig trennenden Scheidelinien angeordnet ist, wodurch die mehrzeilige Detektionsvorrichtung gebildet wird.

2. Herstellungsverfahren der multi-linierten Detektionsvorrichtung nach Anspruch 1, wobei ein elastisches Material auf der zylindrischen Außenfläche des ersten Wälzfräsers zwischen zwei benachbarten Linearschneidern des ersten Wälzfräsers platziert wird und die Spitzen der Linearschneider das elastische Material um einen geeigneten Abstand überschreiten, wobei der geeignete Abstand vorzugsweise kleiner als die Dicke des Teststreifens ist.

3. Herstellungsverfahren der multi-linierten Detektionsvorrichtung nach Anspruch 1, wobei der zweite Wälzfräser mit mindestens zwei ersten rechteckigen Schneidern und zweiten rechteckigen Schneidern in gleichem Abstand auf der zylindrischen Außenfläche davon versehen ist und die gleiche Anzahl von mindestens zwei ersten rechteckigen Schneidern zwischen jeweils zwei benachbarten zweiten rechteckigen Schneidern angeordnet ist; wobei die langen Seiten des ersten und zweiten rechteckigen Schneiders parallel zur Achse der zylindrischen Außenfläche sind; und wobei der erste rechteckige Schneider zwei unvollständig getrennte Schnittlinien bildet und der zweite rechteckige Schneider eine unvollständig trennende Schnittlinie und eine vollständig trennende Schnittlinie bildet.

4. Herstellungsverfahren der multi-linierten Detektionsvorrichtung nach Anspruch 3, wobei zwei Längsseiten des ersten rechteckigen Schneiders, die auf der zylindrischen Außenfläche des zweiten Wälzfräsers angeordnet sind, gezackte Klingen sind, die unvollständig trennende Schnittlinien bilden.

5. Herstellungsverfahren der multi-linierten Detektionsvorrichtung nach Anspruch 4, wobei die gezackten Klingen, die auf der zylindrischen Außenfläche des zweiten Wälzfräsers angeordnet sind, das Siegelfolienband vollständig abschneiden, aber das untere Kartenband nicht vollständig abschneiden oder das untere Kartenband nicht abschneiden.

6. Herstellungsverfahren der multi-linierten Detektionsvorrichtung nach Anspruch 3, wobei eine der langen Seiten jedes zweiten rechteckigen Wälzfräsers auf der zylindrischen Außenfläche des zweiten Wälzfräsers eine lineare Klinge ist, die andere der langen Seiten eine gezackte Klinge ist, wobei die lineare Klinge eine vollständig trennende Schnittlinie bildet und die gezackte Klinge eine unvollständig trennende Schnittlinie bildet.

7. Herstellungsverfahren der multi-linierten Detektionsvorrichtung nach Anspruch 6, wobei die lineare Klinge des zweiten rechteckigen Schneiders sowohl das Siegelfolienband als auch das untere Kartenband vollständig abschneidet und die gezackte Klinge des zweiten rechteckigen Schneiders das Siegelfolienband vollständig abschneidet, aber das untere Kartenband nicht vollständig abschneidet oder das untere Kartenband nicht durchtrennt.

8. Herstellungsverfahren der multi-linierten Detektionsvorrichtung nach Anspruch 1, wobei die Dicke des unteren Kartenbandes von 0,2 mm bis 1,5 mm und die Dicke des Siegelfolienbandes von 0,02 mm bis 0,1 mm reicht.

9. Herstellungsverfahren der multi-linierten Detektionsvorrichtung nach Anspruch 7, wobei die Dicke des unteren Kartenbandes von 0,2 mm bis 1,5 mm und die Dicke des Siegelfolienbandes von 0,02 mm bis 0,1 mm reicht.

## Revendications

1. Procédé de fabrication d'un dispositif de détection multiligne (501), comprenant les étapes suivantes:
A, fournissant une bande inférieure de carte (603), une sangle d'essai (604) et une bande de film d'étanchéité (608), dans laquelle l'épaisseur de la bande inférieure de la carte est supérieure à l'épaisseur de la bande de film d'étanchéité;
B, découper la sangle d'essai en au moins deux bandelettes d'essai (606) disposées côte à côte à l'aide d'une première table de cuisson cylindrique (605), la première plaque de cuisson étant pourvue d'au moins deux fraises linéaires (607) sur la surface extérieure cylindrique de celle-ci, les lames linéaires étant parallèles à l'axe de la surface extérieure cylindrique et ayant des distances égales par rapport à l'axe de la surface extérieure cylindrique, les au moins deux fraises linéaires disposées à intervalles égaux sur la surface extérieure cylindrique de la première fraise-mère ;
C, en plaçant séquentiellement les bandelettes de test coupées sur la bande inférieure de la carte de sorte que les bandes de test soient collées à des positions prédéfinies sur la bande inférieure de la carte;
D, le transfert de la bande de film d'étanchéité vers le haut de la bande inférieure de la carte, le collage de la bande de film d'étanchéité sur la bande inférieure de la carte par chauffage et compression à l'aide d'un procédé de thermoscellage, et l'emballage desdites bandelettes d'essai respectivement dans des cavités étanches à l'air correspondantes entre la bande de film d'étanchéité et la bande inférieure de la carte pour former une bande de liaison fermée (610) autour de chaque bandelette d'essai; et
E, en coupant la bande du film d'étanchéité et la bande inférieure de la carte à l'aide d'une deuxième plaque de cuisson cylindrique (611) pour former des lignes de coupe incomplètement déconnectées (612) et des lignes de coupe complètement déconnectées (613), la ligne de coupe incomplètement déconnectée reliant deux dispositifs de détection adjacents, la ligne de coupe complètement déconnectée séparant deux dispositifs de détection adjacents, et le même nombre d'au moins deux lignes de coupe incomplètement déconnectées disposées entre deux adjacentes déconnexion complète des lignes de coupe, formant ainsi le dispositif de détection multi-lignes.

2. Procédé de fabrication du dispositif de détection multiligne selon la revendication 1, dans lequel un matériau élastique est placé sur la surface extérieure cylindrique de la première table de cuisson entre deux lames linéaires adjacentes de la première plaque de cuisson, et les pointes des lames linéaires dépassent le matériau élastique d'une distance appropriée, la distance appropriée étant de préférence inférieure à l'épaisseur de la bande de test.

3. Procédé de fabrication du dispositif de détection multiligne selon la revendication 1, dans lequel la deuxième table de cuisson est pourvue d'au moins deux premières fraises rectangulaires et d'une seconde fraise rectangulaire équidistantes sur la surface extérieure cylindrique de celle-ci, et le même nombre d'au moins deux premières fraises rectangulaires sont disposées entre chaque seconde fraise rectangulaire adjacente ; les côtés longs des première et deuxième fraises rectangulaires sont parallèles à l'axe de la surface extérieure cylindrique ; Et le premier couteau rectangulaire forme deux lignes de coupe incomplètement déconnectées, et le deuxième couteau rectangulaire forme une ligne de coupe incomplètement déconnectée et une ligne de coupe complètement déconnectée.

4. Procédé de fabrication du dispositif de détection multiligne selon la revendication 3, dans lequel deux longs côtés de la première fraise rectangulaire disposés sur la surface extérieure cylindrique de la deuxième table de cuisson sont des lames dentelées, qui forment des lignes de coupe incomplètement déconnectées.

5. Procédé de fabrication du dispositif de détection multi-lignes selon la revendication 4, dans lequel les lames dentelées disposées sur la surface extérieure cylindrique de la deuxième table de cuisson coupent complètement la bande de film d'étanchéité, mais ne coupent pas complètement la bande inférieure de la carte ou ne coupent pas la bande inférieure de la carte.

6. Procédé de fabrication du dispositif de détection multiligne selon la revendication 3, dans lequel l'un des côtés longs de chaque deuxième couteau rectangulaire sur la surface extérieure cylindrique de la deuxième table de cuisson est une lame linéaire, l'autre des côtés longs est une lame dentelée, la lame linéaire formant une ligne de coupe complètement déconnectée, et la lame dentelée formant une ligne de coupe incomplètement déconnectée.

7. Procédé de fabrication du dispositif de détection multiligne selon la revendication 6, dans lequel la lame linéaire du deuxième couteau rectangulaire coupe complètement la bande de film d'étanchéité et la bande de carte inférieure, et la lame dentelée du deuxième couteau rectangulaire coupe complètement la bande de film d'étanchéité, mais ne coupe pas complètement la bande de carte inférieure ou ne coupe pas la bande de carte inférieure.

8. Procédé de fabrication du dispositif de détection multiligne selon la revendication 1, dans lequel l'épaisseur de la bande inférieure de la carte varie de 0,2 mm à 1,5 mm, et l'épaisseur de la bande du film d'étanchéité varie de 0,02 mm à 0,1 mm.

9. Procédé de fabrication du dispositif de détection multiligne selon la revendication 7, dans lequel l'épaisseur de la bande inférieure de la carte varie de 0,2 mm à 1,5 mm, et l'épaisseur de la bande du film d'étanchéité varie de 0,02 mm à 0,1 mm.
